# EUROPEAN PATENT SPECIFICATION

(11) **EP 2 199 278 B1**
(45) Date of publication and mention of the grant of the patent: **14.09.2011**
(21) Application number: 08172275.3
(22) Date of filing: 19.12.2008
(51) Int. Cl.: C07C 269/04

(54) **Process for the Production of Aromatic Urethanes**
Verfahren zur Herstellung von aromatischen Urethanen
Procédé de production d'uréthanes aromatiques

(43) Date of publication of application: 23.06.2010
(73) Proprietor: Dow Global Technologies LLC, Midland, MI 48674 (US)
(72) Inventor: Tundo, Pietro, 30174 Mestre (VE) (IT); Grego, Sandra, 30030 Salzano (VE) (IT); Rigo, Maurizio, 31022 Preganziol (TV) (IT); Paludetto, Renato, 42015 Correggio (RE) (IT)
(74) Representative: Hull, John Philip

(56) References cited:
- WO-A-01/68590
- WO-A-98/55450
- WO-A-2007/015852
- US-A- 4 268 684
- Z.-H. FU AND Y. ONO: "synthesis of methyl N-phenyl carbamate by methoxycarboxylation of aniline..." JOURNAL OF MOLECULAR CATALYSIS., vol. 91, 1994, pages 399-405, XP002529251 ELSEVIER SEQUOIA, LAUSANNE.

## Description

The present invention relates to a continuous process for the preparation of aromatic urethanes from an organic carbonate and an aromatic amine in the presence of a carbamation catalyst.

Aromatic urethanes are useful intermediates in the preparation of isocyanates. Isocyanates are commonly produced commercially using a reaction between amines and phosgene. However, because of the toxic nature of phosgene, alternative routes are desirable. One way of producing isocyanates which has been discovered is to react an amine with a carbonate to produce a urethane (or carbamate) and then to decompose the urethane thermally to produce the isocyanate.

The reaction of organic carbonates with amines in the presence of a catalyst is well known and is discussed for example in US3,763,217 and US4,268,684. The catalysts used are typically metal salts, and particularly transition metal salts. A preferred catalyst for producing a urethane for decomposition is zinc acetate, as discussed in US6,034,265. Many other catalysts are disclosed in the prior art as being good catalysts for producing high yields and selectivity, such as Lewis acids, for example antimony trichloride or uranyl nitrate, zinc, tin or cobalt salts.

EP0065026 discloses the use of zinc naphthenate, zinc acetate, zinc propionate, zinc octoate as suitable catalysts.

WO99/047493 discloses the use of zinc catalysts such as zinc chloride, zinc acetate, zinc propionate, zinc octoate, zinc benzoate, zinc p-chlorobenzoate, zinc naphthenate, zinc stearate, zinc itaconate, zinc pivalate, zinc phenolate, zinc acetylacetonate, zinc methoxide, lead catalysts like lead acetate and lead octoate, and tin catalyse like stannous chloride, stannous octoate, and mixtures thereof as suitable catalysts. Zinc or copper carbonate hydroxides are disclosed as possible catalysts in US5,688,988.

WO 2007/015852 discloses the use of a heterogeneous catalyst comprising a Group 12-15 metal compound supported on a substrate

The prior art references typically relate to the use of a tank reactor into which the reactants and catalyst are added. Where the catalyst is heterogeneous, it can be removed by filtration and reused. However, inevitably a proportion of catalyst will be lost by filtration.

Even though batch reactors can be used in a semi-continuous or continuous manner, they require the catalyst to be added along with the reactants and then filtered out and recycled, which requires significant time and cost. It would be preferable if the reaction could be undertaken in a manner which did not involve recycling of the catalyst by filtration.

The results of a number of tests carried out by the applicant on various carbamation catalysts indicate that the catalysts are not suitable for use in a continuous fixed bed process as they leach out of the fixed bed, even though they would typically be expected to be insoluble.

Accordingly, in a first aspect of the present invention, there is provided a continuous process for the production of an aromatic urethane from the reaction of an aromatic amine and an organic carbonate comprising the steps of:
passing the aromatic amine and the organic carbonate through a fixed bed reactor containing a zinc carbonate catalyst to produce the aromatic urethane; and
collecting the aromatic urethane.

The process according to the present invention advantageously provides a continuous process in which the catalyst does not need to be filtered out from the reaction product. A continuous process is one in which the reactants flow into the reactor and product out of the reactor throughout the whole of the reaction.

It is preferred that the reaction temperature in the reactor is from 140 to 210°C. It is further preferred that the molar ratio of the organic carbonate and aromatic amine is from 25:1 to 3:1.

The present invention is suitable for use with any organic carbonate and aromatic amine. However, reactants which are of particular importance are dimethylcarbonate and aniline.

In a preferred embodiment, the reactor bed additionally comprises non-catalytic particles such as alumina, silica, silica-alumina, activated carbon, titania, zirconia, and diatomaceous earth.

The term zinc carbonate as used herein is intended to cover any zinc carbonate containing compound of the formula Znₐ(CO₃)_{b}(OH)_{c}(H₂O)_{d} wherein a is from 1 to 4, b and c are from 1 to 7 and d is from 0 to 6.

The organic carbonate can be any alkyl, aryl or alkyl aryl ester of carbonic acid. The ester group can be a an alkyl group with up to 12 carbon atoms, preferably up to 6 carbon atoms, or an aryl group with up to 10 carbon atoms. Suitable organic carbonates include ethylene carbonate, propylene carbonate, styrene carbonate, dimethyl carbonate, diethyl carbonate, dipropyl carbonate, dibutyl carbonate, diisopropyl carbonate, dihexyl carbonate, methyl butyl carbonate, diphenyl carbonate and methyl phenyl carbonate. Dimethyl carbonate (DMC) is particularly preferred.

The aromatic amine can be a primary or secondary amine, with primary amines being preferred. Preferred amines include aniline, 3,4-dichloroaniline, ortho, meta and para-toluidine, 2, 4-xylidene, 3,4-xylidine, 2,5-xylidene, 3-propyl aniline, 4-isopropyl aniline, methyl aniline, ethyl aniline, isopropyl aniline, butyl aniline, heptyl aniline, 4,4'-diamino-diphenyl methane, 2,4,4'-triamino diphenyl ether, 2,6'diaminonaphthalene, 4,4'-bismethylene diphenylamine, 4,4'-methylenedianiline. Particularly preferred is aniline.

In order to obtain complete conversion of the amine to the urethane, the organic carbonate must be present in at least an equivalent stoichiometric ratio. The organic carbonate is preferably present in an excess in order to minimize the side-reactions such as formation of urea. Preferably, the molar ratio of organic carbonate to amine is from 1:1 to 30.1. It is further preferred that the molar ratio is from 3:1 to 25:1 and more preferably from 6:1 to 22:1. It is particularly preferred that the ratio is from 5:1 to 20:1.

The temperature of reaction is from 130 to 220°C, and preferably from 150 to 210°C. It is further preferred that the temperature of reaction is from 180 to 210°C. If the temperature is too low, the reactivity reduces. Conversely, if the temperature of reaction is too high, there is an increase in the amount of side products formed.

The liquid hourly space velocity (LHSV) used in the fixed bed reactor is typically 0.1 to 5.0 hr⁻¹ and more preferably 0.3 to 2.5 her⁻¹.
The reaction is run using a fixed bed reactor. Any standard fixed bed reactor known to the skilled person would be suitable. Examples of fixed bed reactors are disclosed in "Cocurrent Gas-Liquid Downflow in Packed Beds", Chapter 19, of the Handbook of Fluids in Motion (1983) (R. Gupta) and "Multiphase Catalytic Packed Bed Reactors", Catal. Rev. Sci. Eng. 17 (1978) 71-117 (H. Hofmann). A catalyst is packed in a fixed bed reactor by any suitable method known to the skilled person.

In addition to the active catalyst, the bed of the reactor can be packed with other filler materials, such as alumina or silica or the like, to reduce the amount of catalyst present in the bed.

The process should be run under a pressure of 0.5 to 25 MPa, preferably from 0.5 to 10 MPa and more preferably from 1 to 2 MPa.

The skilled person would be aware of methods of setting up a fixed bed reactor to obtain the required reaction conditions and catalyst levels necessary for high reactivity.

The invention will now be further described with reference to the following Examples.

### EXAMPLES

A number of tests were run in a batch trial to find catalysts that were suitably reactive. Tests were undertaken in a 235ml stainless steel pressure vessel equipped with a magnetic stirrer and a temperature control system. 100g of reactants (aniline and dimethylcarbonate) were charged into the reactor and the reactivity as measured over time by sampling the mixture during the reaction. Aniline conversion and carbamate selectivity were calculated from the results. The tests were undertaken using various catalysts.

The reactants used were as follows:
Aniline - Fluka code 10410, purity >99.9%
Dimethylcarbonate - Aldrich code D15292, purity 99, H₂O < 100ppm.

The catalysts used were:
zinc acetate dihydrate from Poletto S.r.1 CAS N. 5970-45-6, MW 219.5;
basic zinc carbonate from Carlo Erba, CAS N. 5260-02-5, MW394.4;
basic lead carbonate from Sigma Aldrich CAS N.1319-46-6, MW775.6;
lead oxide supported on alumina (produced by dissolving a lead salt in water, impregnating the support with the salt until incipient wetness, drying the resulting support for 12 hours at 90°C and calcining at approximately 500°C. The alumina used was Sasol α-alumina No. Z500200 with a particle size of from 0.1 to 0.7µm).

The reaction products were measured using HPLC-UV using nitrobenzene as an internal standard (RP-18 column, eluant water/acetonitrile, gradient from 55/45 to 45/55 in 25 min., flow=1 ml/min.)

The results of the tests are given in Table 1 below:

**Table 1**

| **Example** | **Catalyst** | **Mole% catalyst/mole aniline** | **Wt % aniline based on DMC** | **Temp (°C)** | **Conversion %** | **Carbamate Selectivity %** | **Carbamate +Urea selectivity %** |
|---|---|---|---|---|---|---|---|
| 1 | Zn Acetate | 1.6 | 10 | 160 | 91.9 | 95.1 | 96.0 |
| 2 | Zn Carbonate | 1.6 | 10 | 160 | 97.0 | 93.4 | 96.1 |
| 3 | Pb Carbonate | 1.6 | 10 | 160 | 98.5 | 98.8 | 99.0 |
| 4 | Pb Oxide on Alumina | 1.6 | 10 | 160 | 98.6 | 97.1 | 97.1 |

As can be seen from the results, at the reactant levels and temperatures tested, the lead-containing catalysts show higher conversion and selectivity than those using zinc.

Zinc carbonate, lead carbonate and lead oxide are insoluble or only slightly soluble in water and are generally insoluble in organic solvents at room temperature.

Although batch results are of interest, it is important to see whether the heterogeneous catalysts could be used in a continuous flow reactor. In order to test this, a fixed bed reactor was set up using a 4.15ml reactor of 4.6mm internal diameter and 250mm length, with the catalyst being charged in the reactor and packed manually. The tube containing the catalyst was thermostated with a coiled heating band. The reactant solution of aniline and DMC was fed using a Jasco 880 PU HPLC pump. The products, after the reactor, were cooled using a condenser and were collected after a proportional relief valve, set at P = 1.5 MPa. The reaction products were analysed by HPLC-UV as above.

A solution of aniline (5.0 g, 0.054 moles) and dimethylcarbonate (96.73 g., 1.074 mole), was fed at liquid flow velocity LHSV= 0.72 hr⁻¹ into the continuous flow reactor filled with 2.5 g basic zinc carbonate [ZnCO₃]₂ [Zn(OH)₂]₃ or lead oxide on alumina (4.4g, 11.8% lead).
The reactor was maintained at T=160°C.

After cooling, the raw reaction product was analysed by HPLC (RP-18 column, eluant water/acetonitrile, gradient from 55/45 to 45/55 in 25 min., flow=1 ml/min.). Samples were taken at various times for each catalyst, as shown in Table 2 below:

**Table 2**

| | Ex 5 Zinc Carbonate | | Ex 6 Lead Oxide on Alumina | |
|---|---|---|---|---|
| Time | 5h | 32h | 5h | 20h |
| Aniline | 5.6% | 6.4% | 10.1% | 82.3% |
| Methyl phenyl carbamate | 93.3% | 92.2% | 87.8% | 14.1% |
| N-methyl aniline | 0.6% | 0.7% | 0.8% | 2.4% |
| N-methylcarbamate | 0.0% | 0.0% | 0.2% | 0.0% |
| N,N dimethylaniline | 0.0% | 0.0% | 0.0% | 0.0% |
| Diphenylurea | 0.6% | 0.8% | 1.1% | 1.2% |

From these results it is possible to calculate the following conversion and selectivity values for both catalysts:
- Conversion: 93.3% with respect to the aniline for zinc carbonate after 5 hours and 92.2% after 32 hours.
- Conversion: 89.9% with respect to the aniline for lead oxide after 5 hours and only 17.7% after 20 hours.
- Selectivity with respect to methyl-phenyl carbamate for zinc carbonate: 98.8% after 5 hours and 98.5% after 32 hours.
- Selectivity with respect to methyl-phenyl carbamate for lead oxide: 88.1 % after 5 hours and 79.5% after 20 hours.

Although both catalysts were thought to be insoluble in the reactants and showed very similar properties when trialled batchwise, it can be seen clearly that there is a dramatic drop-off in activity seen in the use of the lead oxide catalyst after only 20 hours. Conversely, using the catalyst in the process of the present invention, the conversion and selectivity remain essentially static over a period of 32 hours. In the case of the lead catalyst, the resultant product solution appeared slightly coloured, indicating a leaching of the catalyst.

Further tests were undertaken using the zinc carbonate catalyst in relation using different flow rates and reaction temperatures in order to optimise the reaction conditions. The results are shown in Table 3 below

**Table 3**

| Example | DMC/Aniline Ratio | LHSV (hr⁻¹) | Temp. °C | Conversion % | Carbamate Selectivity % |
|---|---|---|---|---|---|
| 7 | 20/1 | 0.72 | 160 | 94.0 | 98.6 |
| 8 | 20/1 | 0.72 | 180 | 99.0 | 95.3 |
| 9 | 20/1 | 0.72 | 200 | 99.1 | 96.5 |
| 10 | 10/1 | 0.72 | 160 | 63.5 | 91.9 |
| 11 | 10/1 | 0.72 | 180 | 95.2 | 92.3 |
| 12 | 10/1 | 0.72 | 200 | 97.4 | 94.6 |
| 13 | 10/1 | 1.44 | 180 | 89.3 | 93.6 |
| 14 | 10/1 | 1.44 | 200 | 98.1 | 97.3 |
| 15 | 10/1 | 1.44 | 210 | 96.7 | 95.6 |

As can be seen from the results, a balance of high temperature and high DMC amount gives the best conversion to carbamate. The reaction conditions of a 20/1 ratio, LHSV of 0.72 hr⁻¹ and temperature of 180°C were tested for 132 hours to test for long term degradation and leaching of the catalyst. The results are shown in Table 4 below.

**Table 4**

| | Zinc Carbonate | |
|---|---|---|
| Time | 24h | 132h |
| Aniline | 1.0% | 1.0% |
| Methyl phenyl carbamate | 94.1% | 95.2% |
| N-methyl aniline | 2.3% | 2.2% |
| N-methylcarbamate | 1.6% | 1.0% |
| N,N dimethylaniline | 1.0% | 0.6% |
| Diphenylurea | 0.0% | 0.0% |

As can be seen, the amount of conversion remained the same at 132 hours as for 24 hours (99%) and the selectivity towards carbamate improved over time. The amount of zinc present in the reaction product was measured using atomic absorption spectroscopy analysis. The resulting product contained only 0.8 mg Zn/l which indicates that the zinc carbonate catalyst is stable in a reaction bed. Surprisingly, therefore, zinc carbonate can be seen to be a catalyst which is not only effective in a batch container, but can also be used in a fixed bed reactor without significant leaching of the catalyst. This catalyst is therefore suitable for use in a long term continuous process.

A further test was undertaken using a larger reactor volume of 10.73ml. The amount of catalyst was increased to keep an equivalent amount per unit volume. The flow rate was also increased accordingly. The results showed a comparable conversion rate and carbamate selectivity, suggesting that the continuous process is capable of being scaled up further.

## Claims

1. A continuous process for the production of an aromatic urethane from the reaction of an aromatic amine and an organic carbonate comprising the steps of:
passing the aromatic amine and the organic carbonate through a fixed bed reactor containing a zinc carbonate catalyst to produce the aromatic urethane; and
collecting the aromatic urethane.

2. A continuous process as claimed in Claim 1, wherein the reaction temperature is from 140 to 210°C.

3. A continuous process as claimed in Claim 1 or Claim 2, wherein the molar ratio of the organic carbonate and aromatic amine is from 25:1 to 3:1.

4. A continuous process as claimed in any one of the preceding claims, wherein the organic carbonate is dimethylcarbonate.

5. A continuous process as claimed in any one of the preceding claims, wherein the aromatic amine is aniline.

6. A continuous process as claimed in any one of the preceding claims, wherein the liquid hously space velocity LHSV is from 0.3 to 2.5 hr⁻¹

7. A continuous process as claimed in any one of the preceding claims, wherein the reactor bed additionally comprises non-catalytic particles.

8. A continuous process as claimed in Claim 7, wherein the additional particles comprise at least one of alumina, silica, silica-alumina, activated carbon, titania, zirconia and diatomaceous earth.

## Patentansprüche

1. Kontinuierliches Verfahren für die Herstellung eines aromatischen Urethans aus der Umsetzung eines aromatischen Amins und eines organischen Carbonats mit den folgenden Schritten:
Hindurchleiten des aromatischen Amins und des organischen Carbonats durch einen Festbettreaktor, der einen Zinkcarbonatkatalysator enthält, um das aromatische Urethan zu produzieren; und
Sammeln des aromatischen Urethans.

2. Kontinuierliches Verfahren nach Anspruch 1, wobei die Reaktionstemperatur von 140 bis 210°C beträgt.

3. Kontinuierliches Verfahren nach Anspruch 1 oder Anspruch 2, wobei das Molverhältnis des organischen Carbonats und des aromatischen Amins von 25:1 bis 3:1 beträgt.

4. Kontinuierliches Verfahren nach einem der vorhergehenden Ansprüche, wobei das organische Carbonat Dimethylcarbonat ist.

5. Kontinuierliches Verfahren nach einem der vorhergehenden Ansprüche, wobei das aromatische Amin Anilin ist.

6. Kontinuierliches Verfahren nach einem der vorhergehenden Ansprüche, wobei die stündliche Flüssigkeitsraumgeschwindigkeit (LHSV) von 0,3 bis 2,5 hr⁻¹ beträgt.

7. Kontinuierliches Verfahren nach einem der vorhergehenden Ansprüche, wobei das Reaktorbett außerdem nicht-katalytische Teilchen umfasst.

8. Kontinuierliches Verfahren nach Anspruch 7, wobei die zusätzlichen Teilchen mindestens eines von Aluminiumoxid, Siliciumoxid, Siliciumoxid-Aluminiumoxid, Aktivkohle, Titandioxid, Zirconiumdioxid und Diatomeenerde umfassen.

## Revendications

1. Procédé continu de production d'un uréthane aromatique par réaction d'une amine aromatique et d'un carbonate organique, lequel procédé comporte les étapes suivantes :
- faire passer l'amine aromatique et le carbonate organique dans un réacteur à lit fixe contenant un catalyseur à base de carbonate de zinc, pour produire l'uréthane aromatique ;
- et recueillir cet uréthane aromatique.

2. Procédé continu conforme à la revendication 1, dans lequel la réaction se déroule à une température de 140 à 210 °C.

3. Procédé continu conforme à la revendication 1 ou 2, dans lequel le rapport molaire entre le carbonate organique et l'amine aromatique vaut de 25/1 à 3/1.

4. Procédé continu conforme à l'une des revendications précédentes, dans lequel le carbonate organique est du carbonate de dimé-thyle.

5. Procédé continu conforme à l'une des revendications précédentes, dans lequel l'amine aromatique est de l'aniline.

6. Procédé continu conforme à l'une des revendications précédentes, dans lequel la vitesse spatiale horaire de liquide (VSHL) vaut de 0,3 à 2,5 h⁻¹.

7. Procédé continu conforme à l'une des revendications précédentes, dans lequel le lit du réacteur comprend en outre des particules non-catalytiques.

8. Procédé continu conforme à la revendication 7, dans lequel les particules supplémentaires comprennent des particules d'au moins l'un des corps suivants : alumine, silice, silice-alumine, charbon actif, oxyde de titane, zircone, et terre à diatomées.
